# EUROPÄISCHE PATENTSCHRIFT

(11) **EP 0 716 705 B1**
(45) Veröffentlichungstag und Bekanntmachung des Hinweises auf die Patenterteilung: **22.03.2000**
(21) Anmeldenummer: 94927528.3
(22) Anmeldetag: 01.09.1994
(51) Int. Cl.: C12N 15/81, C12N 15/62

(54) **VERFAHREN ZUR REKOMBINANTEN HERSTELLUNG VON PROTEINEN IN DER HEFE HANSENULA**
METHOD FOR THE RECOMBINANT PREPARATION OF PROTEINS IN HANSENULA YEAST
PROCEDE DE PREPARATION DE PROTEINES PAR RECOMBINAISON DANS LA LEVURE D'HANSENULA

(30) Priorität: 04.09.1993 DE 4329969
(43) Veröffentlichungstag der Anmeldung: 19.06.1996
(73) Patentinhaber: BASF AKTIENGESELLSCHAFT, 67056 Ludwigshafen (DE); Rhein Biotech Gesellschaft für biotechnologische Prozesse und Produkte mbH, 40595 Düsseldorf (DE)
(72) Erfinder: SCHWEDEN, Jürgen, D-67433 Neustadt (DE); BOLLSCHWEILER, Claus, D-69118 Heidelberg (DE); PIONTEK, Michael, D-45136 Essen (DE); WEYDEMANN, Ulrike, D-50825 Köln (DE); JANOWICZ, Zbigniew A., D-40699 Erkrath (DE); STRASSER, Alexander W.M., D-40223 Düsseldorf (DE)
(74) Vertreter: Karau, Wolfgang, Dr.
(86) Internationale Anmeldenummer: EP9402897
(87) Internationale Veröffentlichungsnummer: WO9507356

(56) Entgegenhaltungen:
- WO-A-92/13951
- BIOTECHNOLOGY, Bd.9, März 1991, NEW YORK US Seiten 291 - 295 G. GELLISSEN ET AL 'Heterologous gene expression in Hansenula polymorpha: efficient secretion of glucoamylase' in der Anmeldung erwähnt
- BIOTECH. ADV., Bd.10, 1992 Seiten 179 - 189 G. GELLISSEN ET AL 'High-level expression of foreign genes in Hansenula polymorpha'
- AGRICULTURAL AND BIOLOGICAL CHEMISTRY., Bd.53, Nr.2, 1989, TOKYO JP Seiten 483 - 489 I. YAMASHITA 'The threonine- and serine-rich tract of the secretory glucoamylase can direct beta-galactosidase to the cell envelope'
- BIOCHEMISTRY., Bd.29, 1990, EASTON, PA US Seiten 8998 - 9006 Y. YAMAMOTO ET AL 'Conformational requirement of signal sequences functioning in yeast: circular dichroism and 1H nuclear magnetic resonance studies of synthetic peptides'
- CHEMICAL ABSTRACTS, vol. 116, no. 11, 16. März 1992, Columbus, Ohio, US; abstract no. 100321, P. APRIKYAN ET AL 'Human growth hormone expression and secretion in the methylotropic yeast Hansenula polymorpha' Seite 174 ; & DOKL. AKAD. NAUK SSSR, Bd.321, Nr.2, 1991 Seiten 390 - 394
- GENE, Bd.95, 1990 Seiten 111 - 121 R. DOHMEN ET AL 'Cloning of the Schwanniomyces occidentalis glucoamylase gene (GAM1) and its expression in Saccharomyces cerevisiae'

SEQUENZPROTOKOLL

(1) ALLGEMEINE INFORMATION:

(i) ANMELDER:
(A) NAME: BASF Aktiengesellschaft
(B) STRASSE: Carl-Bosch-Strasse 38
(C) ORT: Ludwigshafen
(E) LAND: Bundesrepublik Deutschland
(F) POSTLEITZAHL: D-67056
(G) TELEPHON: 0621/6048526
(H) TELEFAX: 0621/6043123
(I) TELEX: 1762175170

(A) NAME: Rhein Biotech GmbH
(B) STRASSE: Eichsfelder Strasse 11
(C) ORT: Duesseldorf
(E) LAND: Bundesrepublik Deutschland
(F) POSTLEITZAHL: D-40595
(G) TELEPHON: 0211/9 70 89 0
(H) TELEFAX: 0211/9 70 89 30

(ii) ANMELDETITEL: Verfahren zur rekombinanten Herstellung von
Proteinen in Hefe

(iii) ANZAHL DER SEQUENZEN: 5

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 54 Aminosäuren
(B) ART: Aminosäure
(D) TOPOLOGIE: linear

(ii) ART DES MOLEKÜLS: Peptid

(v) ART DES FRAGMENTS: inneres

(vi) URSPRÜNLICHE HERKUNFT:
(A) ORGANISMUS: Schwanniomyces occidentalis

(xi) SEQUENZBESCHREIBUNG: SEQ ID NO: 1:

Ala Pro Ala Ser Ser Ile Gly Ser Ser Ala Ser Ala Ser Ser Ser Ser
1               5                   10                  15

Glu Ser Ser Gln Ala Thr Ile Pro Asn Asp Val Thr Leu Gly Val Lys
            20                  25                  30

Gln Ile Pro Asn Ile Phe Asn Asp Ser Ala Val Asp Ala Asn Ala Ala
        35                  40                  45

Ala Lys His Pro Leu Glu
    50

(2) INFORMATION ZU SEQ ID NO: 2:

(i) SEQUENZ CHARAKTERISTIKA:
(A) LÄNGE: 33 Basenpaare
(B) ART: Nukleinsaure
(C) STRANGFORM: Einzel
(D) TOPOLOGIE: linear

(ii) ART DES MOLEKÜLS: DNS (genomisch)

(xi) SEQUENZBESCHREIBUNG: SEQ ID NO: 2:

GGGGGGGAAT TCATGATTTT TCTGAAGCTG ATT 33

(2) INFORMATION ZU SEQ ID NO: 3:

(i) SEQUENZ CHARAKTERISTIKA:
(A) LÄNGE: 31 Basenpaare
(B) ART: Nukleinsaure
(C) STRANGFORM: Einzel
(D) TOPOLOGIE: linear

(ii) ART DES MOLEKÜLS: DNS (genomisch)

(xi) SEQUENZBESCHREIBUNG: SEQ ID NO: 3:

GGGGGGCAGC TGCATTAGCA TCGACAGCAG A 31

(2) INFORMATION ZU SEQ ID NO: 4:

(i) SEQUENZ CHARAKTERISTIKA:
(A) LÄNGE: 56 Basenpaare
(B) ART: Nukleinsaure
(C) STRANGFORM: Einzel
(D) TOPOLOGIE: linear

(ii) ART DES MOLEKÜLS: DNS (genomisch)

(xi) SEQUENZBESCHREIBUNG: SEQ ID NO: 4:

GGGGGGCAGC TGCTAAACAC CCTCTGGAAA AAAGAGTTGT TTACACTGAC TGCACT 56

(2) INFORMATION ZU SEQ ID NO: 5:

(i) SEQUENZ CHARAKTERISTIKA:
(A) LÄNGE: 45 Basenpaare

(B) ART: Nukleinsäure
(C) STRANGFORM: Einzel
(D) TOPOLOGIE: linear

(ii) ART DES MOLEKÜLS: DNS (genomisch)

(xi) SEQUENZBESCHREIBUNG: SEQ ID NO: 5:

GGGGGGGTCG ACCCTAGATC TCTATTACTG CAGGTATTCT TCCGG 45

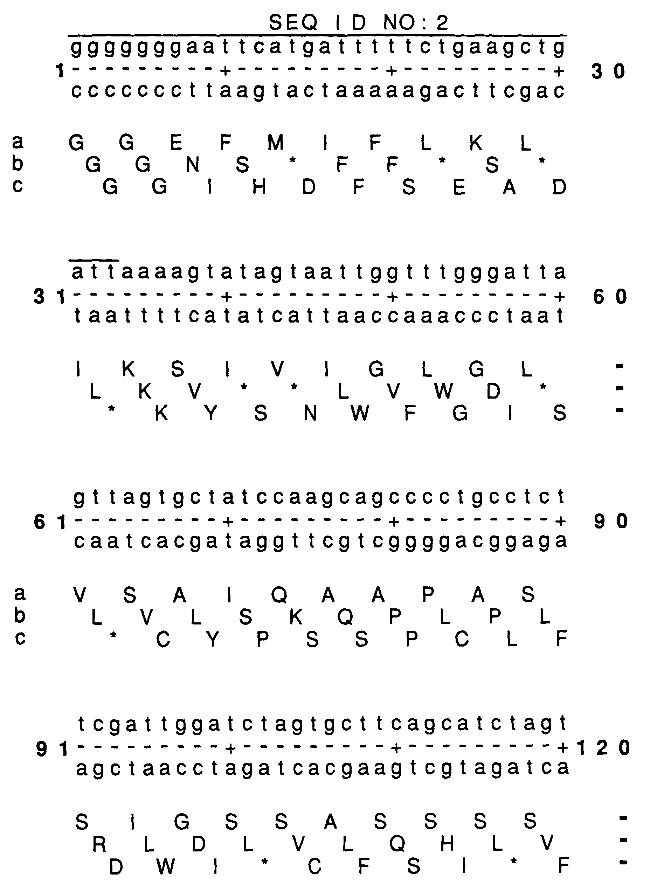

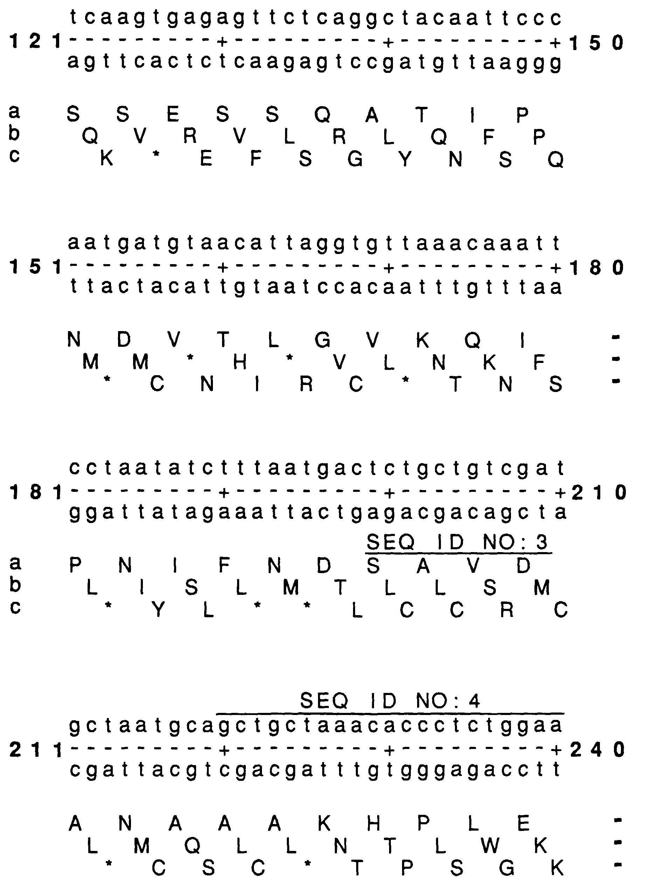

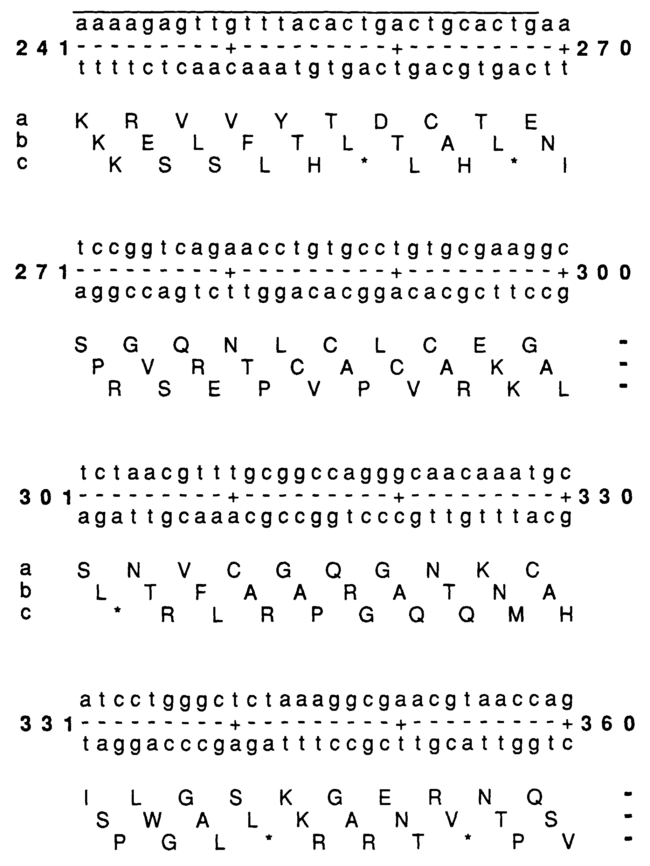

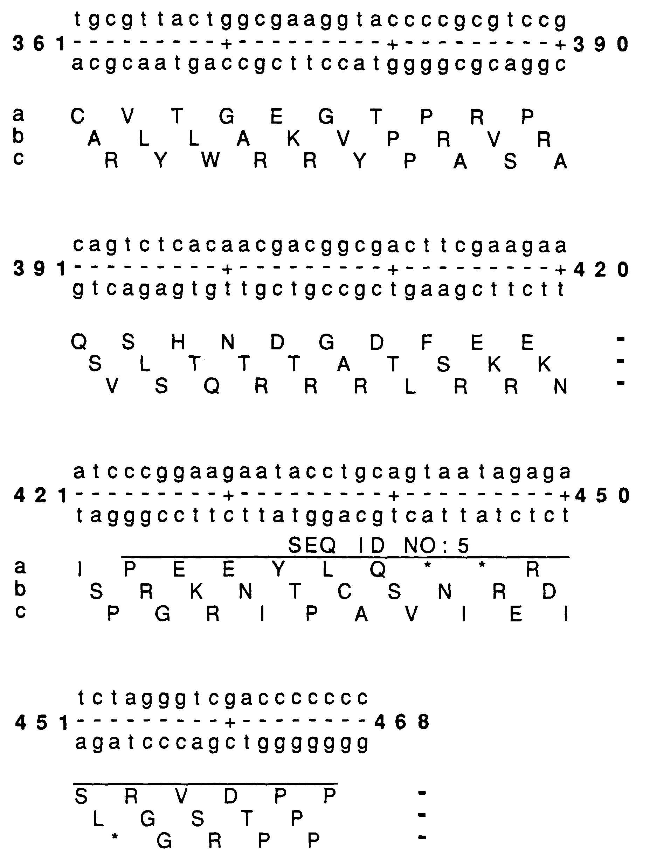

## Beschreibung

Die vorliegende Erfindung betrifft ein Verfahren zur rekombinanten Herstellung von Hefe-fremden Proteinen in der Hefe Hansenula.

Die rekombinante Herstellung von Proteinen in der Hefe Hansenula ist bekannt. In der europäischen Patentschrift EP 173378 wird die rekombinante Herstellung von Proteinen unter Verwendung bestimmter Promotorelemente von MOX oder DAS beschrieben. Jedoch gibt dieses Dokument keine Hinweise, wie eine effektive Sekretion sowie eine korrekte Prozessierung des gewünschten Proteins zu erreichen ist.

Weiterhin ist bekannt, daß in Hansenula polymorpha die Glucoamylase-Leadersequenz (GAM1) aus Schwanniomyces occidentalis als Signalsequenz erkannt wird und es möglich ist, korrekt prozessierte Glucoamylase zu sezernieren (G. Gellissen et al., Biotechnology 9, 291-295, 1991). Diese Signalsequenz führt aber nicht zur Sekretion Hefe-fremder Genprodukte, beispielsweise ist die Sekretion des Proteins Hirudin damit nicht möglich.

Aufgabe der vorliegenden Erfindung war es daher, ein Verfahren zur rekombinanten Herstellung von Proteinen, insbesondere von Hefe-fremden, d.h. heterologen Proteinen, in der Hefe Hansenula bereitzustellen, das für eine Vielzahl von Proteinen die effiziente Sekretion und korrekte Prozessierung gewährleistet.

Diese Aufgabe wird gelöst durch ein Verfahren zur rekombinanten Herstellung von Hefe-fremden Proteinen in der Hefe Hansenula, das dadurch gekennzeichnet ist, daß Hansenula mit einer Expressionskassette transformiert wird, die folgende Strukturelemente codiert enthält:
L - A - P - GEN
wobei
- L:: eine Leadersequenz,
- A:: ein eine alpha-Helix-Struktur erzeugender Adaptor,
- P:: ein Prozessierungssignal und
- GEN:: ein Strukturgen für das gewünschte Protein
darstellen.

Als Leadersequenz L können die Leadersequenzen von allen in Hefe sezernierten Genprodukten verwendet werden, die von Hansenula erkannt werden. Es ist nicht notwendigerweise erforderlich, daß die Leadersequenz von einem Hansenula-Gen stammt. Es sind auch Leadersequenzen von Hefen anderer Gattungen als Hansenula geeignet, beispielsweise Saccharomyces oder Schwanniomyces. Eine für die Erfindung gut geeignete Leadersequenz ist beispielsweise die alpha-Faktor-Leadersequenz aus Saccharomyces cerevisiae (MATα).

Bevorzugt werden als Leadersequenzen solche von stark exprimierten und sezernierten hydrolytischen Enzymen verwendet wie alpha-Amylase, Invertase, saure Phosphatase oder Glucoamylase. Besonders bevorzugt wird die Glucoamylase-Leadersequenz aus Schwanniomyces occidentalis verwendet.

Als Adaptor A sind alle Sequenzen geeignet, die für ein Polypeptid codieren, das eine alpha-Helix-Struktur enthält. Das Vorhandensein einer alpha-Helix-Struktur kann nach dem Algorithmus von Garnier et al. (J. Mol. Biol. 120, 97-120, 1978) ermittelt werden. Besonders leicht läßt sich mit kommerziell erhältlichen Computerprogrammen, die auf diesem Algorithmus basieren, ermitteln, ob eine Polypeptidsequenz eine alpha-Helix-Struktur besitzen sollte.

In der Regel sind alle solche Sequenzen als Adaptor gut geeignet, für die mit dem Computerprogramm "Microgenie" (Beckmann) im Bereich der Prozessierungsstelle A-P-GEN für eine Peptidsequenz von mindestens vier Aminosäuren für ALPHA ein größerer positiver Wert als für die drei anderen Strukturmöglichkeiten (BETA, TURN, COIL) errechnet wird.

Für die erfindungsgemäße Anwendung kann die Länge der Adaptorsequenz A in großen Bereichen variieren. Sie beträgt in der Regel zwischen fünf und einhundert Aminosäuren.

Bevorzugt wird als Adaptorsequenz eine Sequenz der Glucoamylase aus Schwanniomyces occidentalis verwendet, die die Aminosäuren 23-72 (GAM 23-72; Dohmen et al. Gene, 95, 111-121 (1990)) enthält.

Diese Sequenz kann direkt oder besonders bevorzugt nach Verlängerung an C-Terminus um ein bis vier Aminosäuren als Adaptorsequenz verwendet werden. Auch Teile dieser Sequenz, bevorzugt solche die durch N-terminale Verkürzung erhalten werden, sind für das erfindungsgemäße Verfahren gut geeignet.

Man kann beispielsweise auch anhand des oben beschriebenen Computerprogramms die Sequenzbereiche, die besonders zur alpha-Helix-Bildung beitragen, identifizieren und durch Austausch einzelner Aminosäuren noch hinsichtlich der alpha-Helix-Struktur optimieren.

Für die Herstellung von Thrombininhibitoren, insbesondere von Hirudin und Hirudinderivaten nach dem erfindungsgemäßen Verfahren hat sich als Adaptor folgende Sequenz als besonders gut geeignet erwiesen:
GAM 23-72 - His - Pro - Leu - Glu (SEQ ID NO : 1)

Wenn diese Sequenz (=A) mit der Leadersequenz der Glucamylase (GAM 1-22) (=L) kombiniert wird erhält man eine Leader-Adaptor-Sequenz mit der Struktur GAM 1-72 - His - Pro - Leu - Glu aus 76 Aminosäuren, die für das erfindungsgemäße Verfahren besonders vorteilhaft ist.

Das Prozessierungssignal P dient der Spaltung des Propeptids in die reife Form. Üblicherweise wird eine Sequenz aus basischen Aminosäuren als Prozessierungssignal verwendet. Gut geeignet als Prozessierungssignal ist die KEX2-Erkennungsstelle aus S. cerevisiae, die aus dem Dipeptid Lys - Arg besteht und auch von der Hefe Hansenula erkannt wird. Dieses Dipeptid kann auch in duplizierter Form als Prozessierungssignal verwendet werden. Bevorzugt wird als P die Sequenz Lys - Arg.

Als Strukturgen GEN für das herzustellende Protein können heterologe und homologe Gene verwendet werden. Die Gene können aus den entsprechenden Organismen isoliert oder synthetisch hergestellt werden. Bei chemischer Gensynthese kann gewünschtenfalls auch eine Anpassung der Codon-usage an den Produktionsorganismus erfolgen.

Bevorzugt werden als Strukturgene eukaryotische Gene eingesetzt. Das erfindungsgemäße Verfahren gelingt besonders gut für die Herstellung von Thrombininhibitoren, beispielsweise von Hirudin. Auch für die Herstellung von humanen Polypeptiden beispielsweise von Peptidhormonen, Wachstumsfaktoren, Lymphokinen ist dieses Verfahren gut geeignet.

Die oben genannten Strukturelemente werden in bekannter Weise in der Folge L - A - P - GEN in einer Expressionskassette angeordnet. Die Verknüpfung geschieht üblicherweise durch Ligation kompatibler Restriktionfragmente oder durch chemische Synthese.

Die Expressionskassetten können weiterhin eine Reihe von üblichen Regulationssignalen wie Promotoren, RBS-Bindungsstellen, Terminatoren enthalten, die funktionell mit den erfindungsgemäßen Strukturelementen L - A - P - GEN verbunden werden.

Die Expressionskassette kann Bestandteil eines autonom replizierenden oder auch eines integrativen Vektors sein. Die Konstruktion eines Expressionsvektors unter Verwendung der Expressionskassette ist in Beispiel 1 beschrieben.

Die Hefe Hansenula wird mit dem entsprechenden Expressionsvektor, der die Expressionskassette enthält, transformiert. Dies kann beispielsweise nach dem in Beispiel 2 beschriebenen Protokoll geschehen.

Von der so transformierten Hefe werden stabil exprimierende Klone isoliert, die als Produktionsorganismus in dem erfindungsgemäßen Verfahren geeignet sind. Die Produktionsorganismen werden unter üblichen Bedingungen gezüchtet und produzieren je nach gewählten Regulationselementen in konstitutiver oder induzierbarer Weise das gewünschte Protein. Das Protein wird vom Produktionsorganismus in das umgebende Medium sezerniert, von wo es leicht isoliert und gereinigt werden kann.

Die Aufreinigung aus dem Medium geschieht in der Regel, nachdem der Produktionsorganismus abgetrennt worden ist, durch in der Proteinchemie geläufige Reinigungsverfahren.

Das erfindungsgemäße Verfahren liefert korrekt prozessierte reife Proteine ohne die sonst zu beobachtende Fehlprozessierung. Daher führt dieses Verfahren zu einer hohen Ausbeute an reifem Protein und erleichtert die nachfolgenden Reinigungsschritte erheblich. Dieses Verfahren ist daher besonders gut bei der Herstellung von Arzneimitteln auf Basis von Pharmaproteinen einsetzbar.

Die folgenden Beispiele dienen der weiteren Veranschaulichung der Erfindung.

### Beispiel 1

Konstruktion von Vektoren zur sekretorischen Expression von rekombinanten Proteinen aus dem Hefestamm Hansenula polymorpha

In diesem Beispiel wird die Konstruktion von Expressionsvektoren beschrieben, die bei der erfindungsgemäßen Herstellung rekombinanter Proteine in Hansenula polymorpha zur Anwendung kommen. Die dabei verwendete Expressionskassette besteht u.a. aus folgenden Bestandteilen:
- Leader:: Aminosäure 1-22 der Glucoamylase Sequenz aus Schwanniomyces occidentalis (Dohmen et al. Gene, 95, 111-121, 1990)
- Adaptor:: SEQ ID NO: 1
- Prozessierungssignal:: Lys-Arg
- GEN:: Thrombininhibitor-Gen

Ausgehend von der oben angeführten Glucoamylase-Sequenz von Schwanniomyces occidentalis wurde mit Hilfe synthetischer Oligonukleotide und PCR-Amlifikation die GAM-Sequenz von Basenpaar 1 bis 207 (entspricht Aminosäure 1 (Met) bis Aminosäure 69 (Ala) hergestellt; Fig.).

Als Amplifizierungsprimer wurden zwei Oligonukleotide mit den Sequenzen SEQ ID NO: 2 und SEQ ID NO: 3 hergestellt und für die PCR eingesetzt.

Das so entstandene GAM Leader-Adaptor Teilfragment wurde am 5'-Ende mit EcoRI und an 3'-Ende mit PvuII nachgeschnitten.

Zur sekretorischen Herstellung von Hirudin wurde ausgehend vom bekannten Hirudingen mit Hilfe von zwei synthetischen Oligonukleotiden und PCR Amplifikation ein Adaptor-ProzessierungssignalHirudingen (A-P-GEN) hergestellt. Die dazu verwendeten Oligonukleotide hatten die Sequenz SEQ ID NO: 4 und SEQ ID NO: 5.

Das amplifizierte DNA-Fragment wurde ein 5'-Ende mit PvuII und am 3'-Ende mit SalI nachgeschnitten.

Durch Ligation der 3'-Ende PvuII-Stelle des GAM-Leader-Adaptor-Teilfragments mit der 5'-Ende PvuII-Stelle des A-P-GENs sowie durch Ligation dieses Fragments über EcoRI / SalI in pUC18 wurde das Konstrukt fertiggestellt.

Aus diesem Konstrukt wurde wiederum das L-A-P-GEN-Fragment als EcoRI/BgIII-Fragment isoliert und in den entsprechend vorbereiteten H.polymorpha Expressionsvektor pFMD 13025 (Gellissen G. et al., TIBTECH, 10, 413-417, 1992) ligiert. Dabei wird das 5' Ende von L-A-P-GEN an den H.polymorpha-Promotor und das 3' Ende des Fragmentes an den H.polymorpha-Terminator fusioniert. Die Expressionskassette ist nunmehr vollständig und Bestandteil eines Shuttle-Vektors, mit dem zwecks Propagierung sowohl E.coli als auch zwecks Expression des Fremdgens die Hefe H.polymorpha transformiert werden kann.

Die gleiche L-A-P Konstruktion wurde mit dem Gen für den Thrombininhibitor Rhodniin aus Rhodnius prolixus (WO 93/8282) sowie mit dem Gen für den Thrombininhibitor Moubatin aus Ornithodorus moubata (WO 93/9232) fusioniert. Die dabei erhaltenen Expressionskassetten wurden analog zu den Hirudingenfusionen für die Konstruktion von Hansenula polymorpha Expressionsvektoren eingesetzt.

### Beispiel 2

Transformation von Hansenula polymorpha mit den Expressionsvektoren

Wirtsstamm für die Transformation ist eine durch EMS-Mutagenese erhaltene auxotrophe Mutante: ein Stamm mit einer Defizienz für Orotidin-5'-Phosphat-Dehydrogenase (ura⁻). Die Reversionsrate dieser Uracil-Mutante kann vernachlässigt werden.

Kompetente Zellen dieses Stammes erhielt man folgendermaßen (nach Dohmen et al., Yeast 7, 691-692, 1992):

10 ml Hefe-Vollmedium (YPD) wurden mit dem Wirtsstamm beimpft und über Nacht bei 37°C schüttelnd kultiviert. Diese Vorkultur wurde in 200 ml YPD-Medium überimpft und bei 37°C schüttelnd bis zu einer OD_{600 nm} = 0,6 - 1,0 kultiviert. Die Zellen wurden mit 0,5 ml Volumen Lösung A (1 M Sorbitol, 10 mM Bicin pH 8,35, 3 % Ethylenglycol) bei Raumtemperatur gewaschen und anschließend in 0,02 Volumen der Lösung A resuspendiert.

Nach Zusatz von 11 µl DMSO wurden die Aliquots bei -70°C bis zur Transformation gelagert.

Zwecks Transformation wurden 10 µg Plasmid-DNA und 100 µl kalte 0,1 M Calciumchloridlösung direkt zu den gefrorenen kompetenten Zellen gegeben.

Nach raschem Auftauen bei 37°C wurde jeder Transformationsansatz mit 1,0 ml Lösung B (40 % Polyethylenglycol PEG 3350, 200 mM Bicin pH 8,35) eine Stunde bei 37°C inkubiert. Die Zellen wurden anschließend in 1 ml Lösung C (150 mM NaCl, 10 mM Bicin pH 8,35) gewaschen, in 200 µl Lösung C resuspendiert und auf Selektivmedium (YNB-Glucose, Komplementierung der Uracil-Defizienz durch ura⁺-Expressionsplasmide) plattiert. Die Inkubation erfolgte für 3 - 5 Tage bei 37°C.

### Beispiel 3

### Isolierung mitotisch stabiler Klone

Die rekombinanten Expressionsplasmide, die für die Transformation von H. polymorpha eingesetzt wurden, sind autonom replizierend und können spontan in das Hefegenom integrieren. Dabei bilden sie eine multimere Struktur: die Plasmidmonomere sind "head to tail" miteinander verbunden.

Mehrere Kopien der Expressionskassette tragen daher zur Produktion des rekombinanten Genproduktes bei. Die Produktivität eines rekombinanten Stammes ist in weiten Bereichen linear zur Anzahl integrierter Expressionskassetten. Die multimere Integration der Fremd-DNA in das Hefegenom und die Isolierung mitotisch stabiler Klone wurde folgendermaßen erreicht:

Die Transformanden wurden von den Agarplatten mit Selektivmedium in 3 ml entsprechendem Flüssigmedium angeimpft und passagiert, d.h. über einen Zeitraum von 1-2 Wochen wurde immer wieder in frisches YNB-Glucose-Medium überimpft (50 µl in 3 ml Medium, Kultivierungen bei 37°C). Während dieser Passagierung integrierte die Plasmid-DNA in das Hefegenom, so daß dann mitotisch stabile Klone erhalten wurden.

Die mitotische Stabilität wurde folgendermaßen getestet:

Aus der letzen Passagierungskultur in YNB-Glucose-Medium wurde dreimal für 1-2 Tage in Vollmedium (YPD) überimpft und bei 37°C kultiviert. Anschließend wurde die verdünnte Kultur auf Vollmedium und auf Selektivmedium plattiert. Mitotisch stabile Transformanden ergeben auf beiden Medien ungefähr gleiche Kolonienzahl. Es können so mitotisch stabile Subtransformanden isoliert werden (Z.A. Janowicz et al., Yeast 7, 431-443 (1991).

### Beispiel 4

### Expression von Fremdgen

Für Expressionsstudien wurden die passagierten Transformanden in 3ml YNB-Medium mit 1 % Glycerin oder 1 % Methanol angeimpft, um MOX-bzw. FMD-Promotoren zu induzieren. Die Zellen wurden nach zweitägiger Kultivierung bei 37°C abzentrifugiert und der Kulturüberstand auf Fremdprotein getestet (Western-Blot, ELISA, Aktivitätstest).

Mit effizient sezernierenden mitotisch stabilen Transformanden wurden 50 ml synthetisches Medium mit 1,5 % Glycerin im 500 ml Schikanen-Erlenmeyer-Kolben angeimpft und bis zu einer OD_{600 nm} = 10 inkubiert. HPLC-Analysen von entsprechenden Kulturüberständen zeigten, daß die Hirudinvariante bei Verwendung der Sequenz GAM 1-72 - His - Pro - Leu - Glu als Leader-Adaptor vollständig korrekt prozessiert wird.

### Beispiel 5

### Fermentation von rekombinanten Hefe-Stämmen

Die rekomnbinanten Hefestämme wurden in synthetischen Medien (doppelt konzentriertes YNB-Medium 2,8 g/L (Difco) mit 10 g/L Ammoniumsulfat) fermentiert, die zu Beginn der Fermentation entweder komplett vorgelegt worden waren oder im Laufe der Fermentation nachgefüttert wurden.

Als Kohlenstoffquellen wurden Glycerin und Methanol oder Gemische aus Glycerin und Methanol eingesetzt. Die Fermentation wurde mit Glycerin als einziger Kohlenstoffquelle gestartet (≥ 1 % Glycerin Endkonzentration im Fermenter während der Anwachsphase).

Nach der Sterilisation des Mediums wurde mit 1 L Vorkultur so angeimpft, daß sich eine Start OD_{600 nm} von ca. 1 ergab.

Der Fermentationsverlauf war zweiphasig: auf eine Anwachsphase mit höherer Glycerinkonzentration (1 %) folgte eine Produktionsphase mit geringerer Glycerinkonzentration (<0,5 %) oder konstanter Methanolkonzentration (1 %) oder einer Mischung aus Glycerin und Methanol (0,1 - 0,4 % Glycerin und 0,2 - 1,0 % Methanol).

Die Kohlenstoffquelle wurde gegebenenfalls über verschiedene Steuerungsmöglichkeiten (kontinuierlich oder pO2 gekoppelt) nachgefüttert.

Im Verlauf der Fermentation wurde Ammoniumsulfat bis zu einer Endkonzentration von 5 g/L , Thiamin bis zu einer Endkonzentration von 0,1 g/L und Biotin bis zu einer Endkonzentration von 0,3 mg/L zugegeben.

Der pH-Wert der Fermentation wurde durch Zugabe von Ammoniakwasser bei 4,0 konstant gehalten; die Fermentationstemperatur betrug 37°C.

Die so fermentierten rekombinanten Hefe-Stämme lieferten ein zu 100 % korrekt prozessiertes Genprodukt (Hirudin).
- Fig.:: Nukleinsäuresequenz des GAM-Leader-Adaptor-Prozessierungssignal-Hirudingentragments und der davon codierten Polypeptidsequenz (Leserahmen a). Die Position der PCR-Primer ist eingezeichnet.

## Patentansprüche

1. Verfahren zur rekombinanten Herstellung von Hefe-fremden Proteinen in der Hefe Hansenula, dadurch gekennzeichnet, daß Hansenula mit einer Expressionskassette transformiert wird, die folgende Strukturelemente codiert enthält:
L - A - P - GEN
wobei
L: eine Leadersequenz,
A: ein eine alpha-Helix-Struktur erzeugender Adaptor,
P: ein Prozessierungssignal und
GEN: ein Strukturgen für das gewünschte Protein
darstellen.

2. Verfahren nach Anspruch 1, dadurch gekennzeichnet, daß als Leadersequenz L die Leadersequenz der Glucoamylase aus Schwanniomyces occidentalis verwendet wird.

3. Verfahren nach Anspruch 1, dadurch gekennzeichnet, daß als Prozessierungssignal P ein oder mehrfach die Peptidsequenz Lys-Arg verwendet wird.

4. Verfahren nach Anspruch 1 bis 3, dadurch gekennzeichnet, daß als Adaptor A die Aminosäuresequenz SEQ ID NO:1 verwendet wird.

## Claims

1. A process for the recombinant production of proteins which are foreign to yeasts in the yeast Hansenula, which comprises transforming Hansenula with an expression cassette which comprises the following structural elements encoded:
L - A - P - GEN
where
L is a leader sequence,
A is an adaptor producing an alpha-helix structure,
P is a processing signal and
GEN is a structural gene for the required protein.

2. A process as claimed in claim 1, wherein the leader sequence of the glucoamylase from Schwanniomyces occidentalis is used as leader sequence L.

3. A process as claimed in claim 1, wherein the peptide sequence Lys-Arg is used one or more times as processing signal P.

4. A process as claimed in any of claims 1 to 3, wherein the amino acid sequence SEQ ID NO:1 is used as adaptor A.

## Revendications

1. Procédé de préparation recombinante de protéines étrangères à des levures, dans la levure hansénula, caractérisé par le fait que l'hansénula est transformée avec une cassette d'expression qui contient sous forme codée les éléments structurels suivants :
L - A - P - GEN
dans laquelle
L : est une séquence leader,
A : est un adaptateur générant une structure à hélice alpha,
P : un signal de traitement, et
GEN : un gène structurel pour la protéine souhaitée.

2. Procédé selon la revendication 1, caractérisé par le fait qu'on utilise comme séquence leader L la séquence leader de la glucoamylase issue de la schwanniomyces occidentalis.

3. Procédé selon la revendication 1, caractérisé par le fait qu'on utilise comme signal de traitement P, à une ou plusieurs reprises, la séquence peptidique Lys-Arg.

4. Procédé selon les revendications 1 à 3, caractérisé par le fait qu'on utilise comme adaptateur A la séquence d'acides aminés SEQ ID NO:1.
